# EUROPEAN PATENT SPECIFICATION

(11) **EP 3 840 818 B1**
(45) Date of publication and mention of the grant of the patent: **15.05.2024**
(21) Application number: 19773921.2
(22) Date of filing: 09.08.2019
(51) Int. Cl.: A61M 35/00

(54) **FLEXIBLE APPLICATOR HANDLE REGION**
GRIFFBEREICH FÜR EINEN FLEXIBLEN APPLIKATOR
RÉGION DE MANIPULATION D'APPLICATEUR SOUPLE

(30) Priority: 21.08.2018 US 201862720694 P
(43) Date of publication of application: 30.06.2021
(73) Proprietor: Solventum Intellectual Properties Company, Maplewood, MN 55144 (US)
(72) Inventor: DOMBROWSKI, Alan R., Saint Paul, Minnesota 55133-3427 (US); ASMUS, Robert A., Saint Paul, Minnesota 55133-3427 (US)
(74) Representative: Mathys & Squire
(86) International application number: PCT/IB2019/056799
(87) International publication number: WO 2020/039296

(56) References cited:
- WO-A1-95/09018
- WO-A1-2005/030297
- US-A1- 2015 231 380
- US-B1- 6 283 933

## Description

### TECHNICAL FIELD

The present disclosure relates to an applicator for applying a liquid to a surface. In particular, the present disclosure relates to an applicator including a handle having a flexible region.

### BACKGROUND

Barrier products are used to protect the skin of patients who suffer from a variety of conditions including, for example, urinary and fecal incontinence, skin occlusion, ileostomy and colostomy. The presence of high moisture and corrosive enzymes from intestinal fluids particularly can lead to devastating breakdown of the skin, which can lead to fungal infections and denuding and erosion of the skin. Some of the products which can be used to protect the skin from these challenges include cyanoacrylate monomer solutions, solutions that require special storage conditions due to their tendencies to degrade upon exposure to ambient conditions.

One method of maintaining the functionality of solutions including cyanoacrylate monomers is to seal such solutions in a container, such as a glass ampoule that can be crushed to release the solution shortly before use. Applicators for dispensing small aliquots of solution, *e.g.,* 0.5 mL to 2 mL, which are particularly desirable when the area to be covered is less than 30 square inches (200 cm²) typically employ glass ampoules placed into plastic tubes with foam attached to them and do not have levers for crushing the ampoule. To provide for a safe device, a suitably rigid and/or thick plastic wall material must be used to form the plastic tube, but since the tube is generally pinched between the fingers to activate, these devices may be difficult for users to activate, especially with a single hand. Documents US6283933 and WO2005/030297 disclose known hand-held applicators provided with a flexible hollow section to enable an ampoule contained inside to be squeezed and broken due to pressure applied by the user over said flexible section.

### SUMMARY

The disclosed applicator includes a handle having an activation region that is sufficiently flexible so that an ampoule inside the applicator body may be readily fractured when the applicator is held in a single hand, while at the same time providing adequate protection to the user from injury by preventing ampoule shards from piercing the applicator body.

In one aspect, the applicator comprises a hollow body. The hollow body includes a distal portion, a proximal portion, an outer surface, an inner surface, and an activation region. The hollow body includes two longitudinal grooves on at least one of the outer surface and the inner surface in the activation region. The hollow body has a first radial groove in the activation region that is on at least one of the outer surface and the inner surface.

Features and advantages of the present disclosure will be further understood upon consideration of the detailed description. The invention is defined in claim 1. Preferable embodiments are defined in the dependent claims.

### BRIEF DESCRIPTION OF THE DRAWINGS

**FIG.** 1 shows a perspective side view of an applicator including an embodiment of an activation region of the present disclosure.
FIG. 2 shows a longitudinal cross-section of the applicator of FIG. 1.
**FIG.** 3 shows a radial cross-section through an applicator activation region having two longitudinal grooves in the outer surface where the grooves are 180° apart.
**FIG.** 4 shows a radial cross-section through an applicator activation region having two longitudinal grooves in the inner surface where the grooves are 180° apart.
**FIG.** 5 shows a radial cross-section through an applicator activation region having two sets of opposing longitudinal grooves in the outer and inner surfaces where the grooves are 180° apart.
**FIG.** 6 shows a radial cross-section through an applicator activation region having a radial groove in the outer surface of the applicator.

Repeated use of reference characters in the specification and drawings is intended to represent the same or analogous features or elements of the disclosure. It should be understood that numerous other modifications and embodiments can be devised by those skilled in the art, which fall within the scope of the disclosure. The figures may not be drawn to scale.

### DETAILED DESCRIPTION

### Definitions:

The term "a", "an", and "the" are used interchangeably with "at least one" to mean one or more of the elements being described.

The term "and/or" means either or both. For example, "A and/or B" means only A, only B, or both A and B.

The terms "including," "comprising," and "having," and variations thereof, are meant to encompass the items listed thereafter, and equivalents thereof, as well as additional items.

The terms "polymer" and "polymeric material" refer to both materials prepared from one monomer such as a homopolymer or to materials prepared from two or more monomers such as a copolymer, terpolymer, or the like. Likewise, the term "polymerize" refers to the process of making a polymeric material that can be a homopolymer, copolymer, terpolymer, or the like. The terms "copolymer" and "copolymeric material" refer to a polymeric material prepared from at least two monomers.

The terms "longitudinal" and "axial" are used to refer to a direction or axis that is generally parallel to a central longitudinal axis of an applicator and generally parallel to the overall direction of substance flow.

The terms "lateral" and "transverse" are used to refer to a direction or axis that is perpendicular to the central longitudinal axis the longitudinal direction.

The terms "vertical" and "normal" are used to refer to a direction or axis that is normal to both the longitudinal and lateral directions (or axes).

The term "proximal" and "distal" are used to represent longitudinal or axial directions, relative to a user (such as, for example, a medical practitioner) using or holding the applicator. That is, the term "distal" is used to refer to the direction away from the medical practitioner (and toward a surface (such as, for example, a skin surface) to be treated, *i.e*., to which the applicator will apply a substance); and the term "proximal" is used to refer to the direction toward the user (and away from the surface to be treated). For example, the distal end of an applicator is configured to be directed toward, or even pressed against, the surface to be treated, while the proximal end extends away from the surface and toward the user. Similarly, the distal end of any portion or component of an applicator is configured to be directed or oriented toward the surface to be treated and is oriented toward the distal end of the applicator or forms or defines the distal end of the applicator. In addition, the proximal end of any portion or component of an applicator is configured to be directed or oriented away from the surface to be treated and is oriented toward the proximal end of the applicator or forms or defines the proximal end of the applicator.

It is often desirable to apply substances to surfaces. In the medical field, single-use applicators may be used to apply medical products to the skin or other tissues. Medical preparations such as, for example, skin barrier products, antiseptics, adhesion enhancing products, adhesive tape trauma protectants, and pharmaceutical agents such as analgesics may be applied as, for example, liquid solutions containing one or more substances. These solutions may be applied with saturated sponges that are attached to a blade or held with forceps. These sponges are often saturated by soaking them in open pans of solution. Sometimes, sponges with attached handles are provided in a plastic or aluminum foil laminate pouch containing enough liquid to saturate the sponges. In some products the sponges are supplied dry in a sterile "kit" with the antiseptic solutions provided in relatively thin-walled polyethylene bottles. These bottles generally have wall thickness less than about 500 microns. While inexpensive, these techniques are messy and offer little control over inadvertent dripping of the solution into areas where it is undesired.

Alternatively, devices have been developed in an attempt to prevent solution from dripping associated with these techniques, and to reduce the time required for application of the antiseptic solution. For example, liquid applicators that hold the liquid in a frangible ampoule and require additional elements to crush the ampoule and release the liquid have been developed. However, existing applicators are often complex to construct, may be difficult or cumbersome to use, and may deliver far more solution than is required for a particular treatment, particularly when a relatively small area of tissue requires substance application.

Treatment of small areas of skin with a solution is needed in some situations, such as, for example, in the application of skin barrier products to persons with ostomies, including ileostomies and colostomies. Treatment of these smaller areas may present challenges not encountered when larger treatment areas are involved. For example, because the treatment area is small, an applicator containing a small volume of solution is preferred in order to minimize wasting what may be a costly solution. Moreover, for conditions such as ostomies, such products may be applied in a nonclinical setting by the person with the condition, creating a need for a simple and easy-to-use applicator that can reliably deliver less than 5 mL (*e.g*., 0.2 - 2 mL) of a solution to a surface in need of treatment.

Applicators that can hold a small amount of substance (*e.g.*, less than 5 mL), that allow for sterilization processes, *e*.*g*., ethylene oxide exposure, and that may be activated and ergonomically used with one hand are needed for certain applications. The present disclosure provides an applicator that, *inter alia*, includes a handle having an activation region that is sufficiently flexible so that the ampoule inside the applicator body may be readily fractured when the applicator is held in a single hand, while at the same time providing adequate protection to the user from injury by preventing ampoule shards from piercing the applicator body.

The present disclosure provides an applicator for applying a substance to a surface, the applicator including a frangible ampoule and a hollow body, where the hollow body includes a distal portion, a proximal portion, an exterior surface, an interior surface, and an activation region. The hollow body includes at least two longitudinal grooves on at least one of the exterior surface and the interior surface in the activation region. The hollow body further includes a first radial groove in the activation region that is on at least one of the exterior surface or the interior surface.

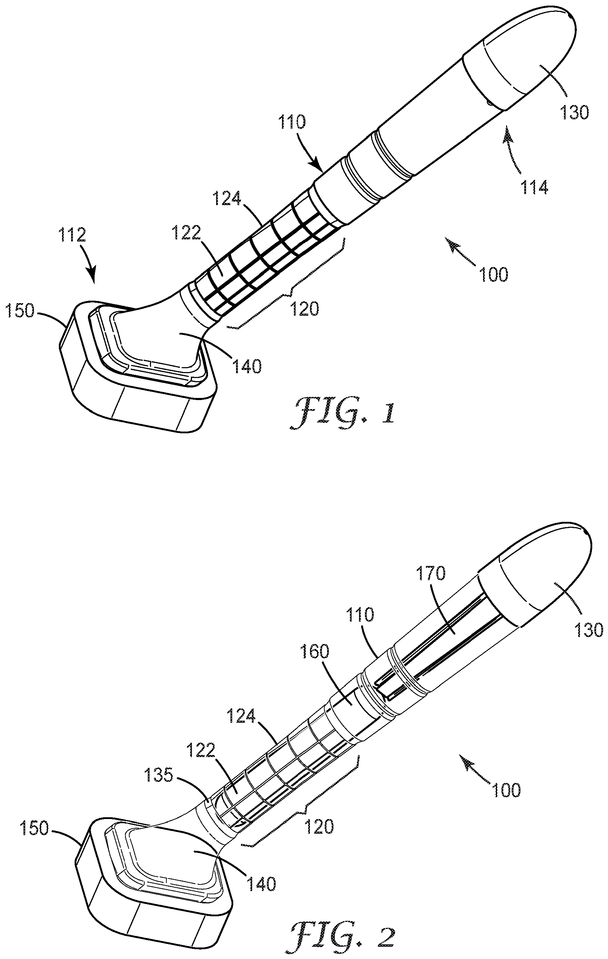

An exemplary applicator **100** according to one embodiment of the present disclosure is illustrated in **FIG. 1****.** The applicator **100** includes a hollow body **110** and a frangible ampoule **160.** Referring to **FIG. 1**, the hollow body **110** includes a distal portion **112**, a proximal portion **114**, an inner surface **116** and an outer surface **118.** The hollow body **120** has a generally frustoconical geometry and may taper as shown along the longitudinal axis of the hollow body **110** from the proximal portion **114** to the distal portion **112.** The taper may facilitate gripping of the applicator **100** in the hand during use. The hollow body **110** includes an activation region **120.**

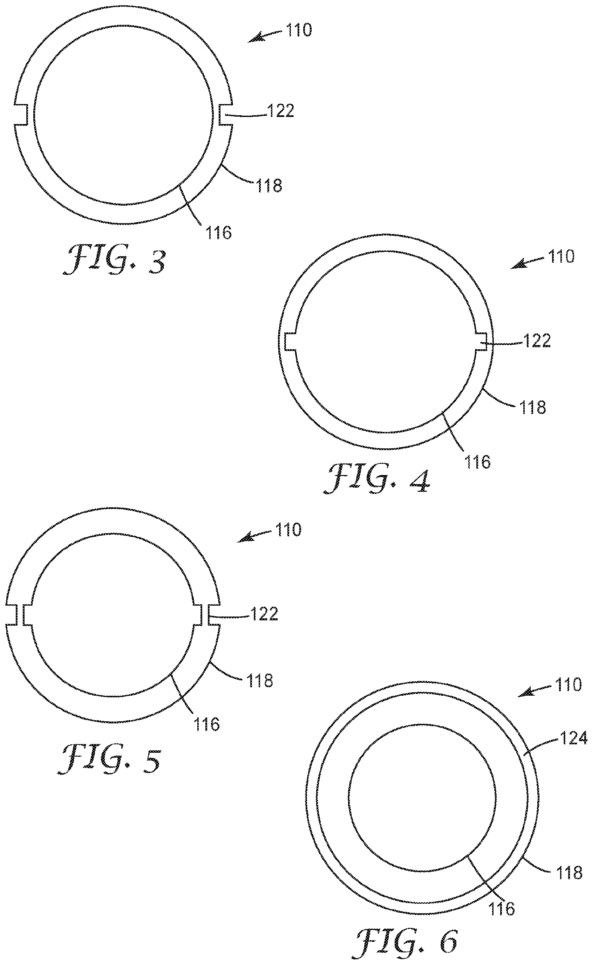

The activation region **120** includes at least two longitudinal grooves **122** on at least one of the inner surface **116** and the outer surface **118** of the activation region. **FIG. 3** shows a radial cross-section through an applicator **100** activation region **120** having two longitudinal grooves **122** in the outer surface **118** where the grooves are spaced 180° apart. In some embodiments, the two longitudinal grooves **122** in the outer surface **118** may be spaced less than 180° apart, such as, for example, 140° apart, 120° apart, 90° apart, 45° apart 30° apart, or 20° apart.

In some embodiments, and as shown in **FIG. 4**, the activation region **120** may have two longitudinal grooves **122** in the inner surface **116** where the grooves are spaced 180° apart. In some embodiments, the two longitudinal grooves 122 in the inner surface 116 may be spaced less than 180° apart, such as, for example, 140° apart, 120° apart, 90° apart, 45° apart 30° apart, or 20° apart.

In some embodiments, and as shown in FIG. 5, the activation region 120 may have two sets of opposing longitudinal grooves 122 in the outer surface 118 and inner surface 116 where the sets of grooves 122 are 180° apart. In some embodiments, the two sets of opposing longitudinal grooves 122 in the outer surface 118 and inner surface 116 may be spaced less than 180° apart, such as, for example, 140° apart, 120° apart, 90° apart, 45° apart 30° apart, or 20° apart.

In some embodiments, the activation region 120 may include more than two longitudinal grooves 122. For example, as shown in FIGS. 1 and 2, the activation region 120 may include six longitudinal grooves 122, *i.e*.*,* three grooves 122 on each side of the activation region 120.

In the present invention, the longitudinal grooves 122 have a width and depth sufficient to impart the desired flexibility to the activation region 120 while not reducing the thickness of the material forming the hollow body 110 to the point where it is vulnerable to puncture. In one embodiment, the hollow body 110 may be formed from a polymer (*e.g*., linear low-density polyethylene) having a thickness of 0.04 inch to 0.020 inch (*e.g.,* 0.028 inch), where the activation region 120 may include a longitudinal groove 122 having a width of 0.001 inch to 0.1 inch (*e.g*., 0.010 inch), and a depth of 0.001 inch to 0.014 inch *(e.g.,* 0.004 inch).

The hollow body 110 further includes a radial groove 124 in the activation region 120 that is on at least one of the inner surface 116 and the outer surface 118 of the activation region. FIG. 6 shows a radial cross-section through an applicator 100 activation region 120 having a radial groove 124 in the outer surface 118 of the applicator 100, wherein the radial groove 124 extends 180° around the body. In some embodiments, the radial groove 124 may extend less than 180° around the body, such as, for example, 160° or less, 150° or less, 140° or less, 130° or less, 120° or less, 110° or less, 100° or less, 90° or less, 80° or less, 70° or less, 60° or less, 50° or less, 40° or less, or 30° or less around the body. Commonly the radial groove 124 will intersect one or more of the longitudinal grooves 122.

The radial groove 124 has a width and depth sufficient to impart the desired flexibility to the activation region 120 while not reducing the thickness of the material forming the hollow body 110 to the point where it is vulnerable to puncture. In one embodiment, the hollow body 110 may be formed from a polymer *(e.g.,* linear low-density polyethylene) having a thickness of 0.04 inch to 0.020 inch *(e.g.,* 0.028 inch), where the activation region 120 may include a radial groove 124 having a width of 0.001 inch to 0.1 inch *(e.g.,* 0.010 inch), and a depth of 0.001 inch to 0.014 inch *(e.g.,* 0.004 inch). In some embodiments, the longitudinal grooves 122 and the radial groove 124 may have the same width and depth dimensions. In some embodiments, the longitudinal grooves 122 and the radial groove 124 may have different width and/or depth dimensions.

The hollow body 110 is configured to retain a frangible ampoule 160. Application of a force to the activation region 120 desirably results in the fracture of the frangible ampoule 160 that is contained in the hollow body **110.** The force may be applied by, for example, the user holding the applicator along the palm of his/her hand and squeezing the applicator between a finger and thumb of that hand in the activation region **120.**

Referring to **FIG. 2**, frangible ampoule **160** is positioned within hollow body **110** such that at least a portion of the frangible ampoule **160** is located in the activation region **120** so that when the activation region **128** is compressed the frangible ampoule **160** is fractured. The frangible ampoule **160** may be hermetically sealed and can contain a solution. The frangible ampoule **160** may desirably act as a protective barrier to contact between the solution contained therein and the outer environment. For example, the frangible ampoule **160** may be resistant to environmental changes encountered by the applicator **100** during manufacture, transport, and use of the applicator, such as, for example, the vacuum and chemicals, *e.g.*, ethylene oxide, that the applicator may be exposed to during a sterilization process, conditions that could damage or destroy the solution inside the frangible ampoule **160.**

Commonly, the frangible ampoule **160** may be formed of a brittle material, such as, for example, a glass. Suitable brittle materials for forming the frangible ampoule **160** include, for example, a soda-lime glass, a borosilicate glass, an onion skin borosilicate glass, a polymer, a ceramic, and combinations thereof. Such materials are desirably brittle and will fracture when compressed. This is in contrast to relatively flexible materials that would deform when compressed but which must be punctured to release the substance inside. Typically, the substance contained within the frangible ampoule **160** is released by applying external force to the applicator **100** in the activation region **120** sufficient to shatter the frangible ampoule **160.** In some embodiments, score lines or other features that provide local areas of weakness in the brittle material may be included to control breaking and/or reduce the force required to break the frangible ampoule **160.**

The size and shape of the frangible ampoule **160** is selected to be compatible with the dimensions of the hollow body **110** and the desired volume of substance to be applied. For example, for use in preparation for a small surgical procedure, the amount of substance in the ampoule **160** should generally be sufficient to cover an area of, such as, for example, about 10 square centimeters. For larger surgical procedures, the amount of substance in the ampoule **160** may need to be sufficient to cover at least the torso of a large person, such as, for example, at least about 500-600 square centimeters. The size of the frangible ampoule **160** may be selected to be appropriate for the volume of substance to be delivered in order to minimize product costs and waste. In addition, smaller frangible ampoules **160** may result in products with longer shelf lives. As a nonlimiting example, it has been found that solutions containing cyanoacrylate adhesives have longer shelf lives in small vials compared to larger vials even when the vials are flushed with an inert gas before sealing. Without being bound by theory, it is believed that the smaller interior surface area of a smaller frangible ampoule has fewer sites on the interior surface of the glass that can initiate polymerization of the cyanoacrylate, thereby extending the shelf life of the product.

In some embodiments, the frangible ampoule **160** is selected to apply a substance to a surface having an area such as, for example, about 5 cm² to about 200 square cm². In some embodiments, use of a smaller frangible ampoule **160** permits a frangible ampoule **160** having a thinner wall to be used, thus increasing the ease with which a frangible ampoule **160** may be fractured. In some embodiments, the frangible ampoule **160** is configured to contain, for example, from about 0.2 mL to about 2 mL *(e.g.,* 1 mL) of a substance.

The frangible ampoule **160** contains the substance to be dispensed. Generally, a wide variety of substances can be contained within the frangible ampoule **160**, with the selection of the substance influencing the selection of the materials used to construct the frangible ampoule and other parts of the applicator **100,** as would be readily understood by one of ordinary skill in the art. Substances contained in the frangible ampoule may include, for example, liquids, gels, suspensions, and pastes. In some embodiments, the substance contained in the ampoule **160** is a liquid composition that includes a volatile carrier and an active agent, such as, for example, an antiseptic agent or a pharmaceutical agent. In some embodiments, the applicator**100** may be particularly useful in dispensing substances having viscosities at room temperature of less than about 10,000 cps. In other embodiments, the applicator may be particularly useful in dispensing substances having viscosities less than about 500 cps.

In some embodiments, the frangible ampoule **160** may contain an antiseptic preparation. Examples of suitable antiseptic preparations include those described in U.S. Pat. No. 4,584,192 and those described in U.S. Pat. No. 4,542,012. Other useful fluids include antiseptic preparations, such as, for example, iodophoric skin tinctures, such as DURAPREP^{™} Surgical Solution, commercially available from 3M Company, Saint Paul, Minnesota, USA. In some embodiments, the ampoule **160** may be filled with a composition that includes an antimicrobial agent such as iodine, an iodine complex (such as, for example, iodophors), chlorhexidine, chlorhexidine salts (such as, for example, chlorhexidine digluconate and chlorhexidine diacetate), or combinations thereof. Other exemplary antimicrobial agents include C2-C5 lower alkyl alcohols, fatty acid monoesters of glycerin and propylene glycol, polymers that include a (C12-C22) hydrophobe and a quaternary ammonium group, polyquaternary amines (such as, for example, polyhexamethylene biguanide), quaternary ammonium silanes, silver, silver salts (such as silver chloride), silver oxide and silver sulfadiazine, methyl, ethyl, propyl and butyl parabens, octenidine, peroxides (such as, for example, hydrogen peroxide and benzoyl peroxide), and the like, as well as combinations thereof. In other embodiments, the frangible ampoule may contain, for example, medical preparations such as skin barrier products, adhesion enhancing products, adhesive tape trauma protectants, and pharmaceutical agents such as analgesics. As a nonlimiting example, the frangible ampoule may contain a skin barrier product, such as CAVILON^{™} Advanced Skin Protectant, commercially available from 3M Company, Saint Paul, Minnesota, USA. In some embodiments, the contents of the frangible ampoule **160** include a cyanoacrylate monomer.

Referring to **FIG. 2****,** the applicator **100** further includes filter **135,** located between the frangible ampoule **160** and a head **140,** the head **140** including an absorbent component **150.** The filter **135** may be integrally molded to the head **140** and is provided with holes so that fluids can readily pass through it. When the frangible ampoule **160** is fractured, the filter **135** prevents shards of the ampoule **160** from reaching the absorbent component **150.** Thus, the filter **135** is made sufficiently thick so as not to be pierced by the shards of the frangible ampoule **160** after the frangible ampoule **160** is broken.

The hollow body **110** may optionally include one or more ribs on the inner surface **126** of the hollow body **110.** The ribs may extend longitudinally along all or part of the length of the hollow body **110** from the filter **135** to the proximal portion **114** of the body. When force is applied to activation region **120,** *e.g.,* by the hand of an operator wishing to coat a surface with the solution contained in the frangible ampoule **160,** the ribs may act as stress concentrators, thereby decreasing the amount of force required to fracture the frangible ampoule **160.**

The applicator further comprises cap **130.** In some embodiments, and as shown in **FIG. 2****,** the applicator optionally further comprises a spacer **170.** Examples of suitable applicators **100** having a cap **130** and spacer **170** include those described in U.S. Pat. App. 62/720,682 filed August 21, 2018.

The distal end of the spacer **170** may have concave curvature. The concave curvature may be selected to complement the convex curvature of an end of an exterior surface of the frangible ampoule **160.** That is, the radius of curvature of the exterior end surface of the frangible ampoule **160** and the radius of curvature of the distal end of the spacer are approximately equal. Thus, the length of spacer **170** and the curvature of its distal end may be selected to maintain the frangible ampoule **160** in a desired position, such as, for example, maintaining the frangible ampoule in the activation region **120** of the body **110.**

In some embodiments, the cap **130** and the spacer **170** may be formed as a unitary piece, such as by, for example, injection molding. In other embodiments, the cap **130** and the spacer **170** may be formed as separate components which could be joined by means known in the art, such as, for example, with an adhesive, a heat seal, a spin weld, an ultrasonic weld, and combinations thereof, or the cap **130** and the spacer **170** could remain as separate components in the assembled applicator **100.**

The cap **130** is adapted to be attached to the hollow body **110** by means of a snap fit. This may be accomplished by providing a retention ring (not shown) inwardly projecting from side walls adjacent to the major orifice of the cap **130** and which has a smaller inside diameter than the outside diameter of the outwardly projecting ridge of an attachment feature near the end of the proximal portion of the body. When the applicator is assembled, the retention ring of the cap is snapped over the outwardly projecting ridge of the attachment feature on the body.

To avoid creating a vacuum and restricting flow through the applicator **100,** a means of maintaining atmospheric pressure in the device is preferably employed. One or more vents in the body functions to aspirate air into the internal volume of the applicator as the substance flows into the absorbent component to maintain atmospheric pressure within the device and prevent "air locking."

Generally, the applicators 100 of the present disclosure can be made by known methods. In some embodiments, injection molding may be used. A variety of materials may be used to form the applicators. In some embodiments, applicators **100** formed of low flexural modulus materials such as low-density polyethylene may be used. For example, in some embodiments, materials having a flexural modulus of no greater than 500 Mpa, *e.g.,* no greater than 400 MPa, or even no greater than 350 MPa may be used.

The applicator body **110,** cap **130,** head **140,** and optional spacer **170** when present may be molded from polymers such as, for example, polyethylenes, linear low density polyethylenes, medium density polyethylenes, high density polyethylenes, branched polyethylenes, polypropylenes, and combinations thereof. In some embodiments, the applicator body **110** may be molded from a combination of linear low-density polyethylene and medium density polyethylene.

The absorbent component **150** may be formed of any suitable porous substance such as, for example, a sponge, a woven material, a nonwoven material, a screen, a mesh, and combinations thereof. Materials suitable for use as the absorbent component **150** may include, for example, polyester polyurethane and polyester polyether open-cell foams. The absorbent component **150** may be attached to the head **140** at the distal end **112** of the hollow body **110** and may, among other functions, aid in control of the flow rate and distribution of the contents of the frangible ampoule **160** after the contents have been released from the frangible ampoule **160.**

In some embodiments, the absorbent component **110** may be attached to the head **140** at the distal end **112** of the hollow body **110** by, for example, hot plate welding. In other embodiments, the absorbent component 110 may be attached to hollow body **120** by other means, such as, for example, an adhesive, a spin weld, an ultrasonic weld, or combinations thereof.

A substance, such as those described above, may be applied to a surface with an applicator as described in the present disclosure by releasing the substance from the frangible ampoule, *e.g*., the user holding the applicator along the palm of his/her hand and squeezing the applicator between a finger and thumb of that hand in the activation region such that the substance flows into the absorbent component when the frangible ampoule is fractured, and then applying the substance to a surface by pressing the absorbent component to the surface.

In some embodiments, the surface comprises mammalian tissue such as, for example, skin, bone, cartilage, enamel, dentin, cementum, dental pulp, gum tissue, mucous membrane, organ tissue, epithelial tissue, ocular tissue, tympanic tissue, connective tissue, muscular tissue, nervous tissue, and combinations thereof. In some embodiments, the mammalian tissue may include a perforation such as, for example, incision, an abrasion, a laceration, a puncture, an orifice, and combinations thereof.

Various modifications and alterations of this invention will become apparent to those skilled in the art without departing from the scope of this disclosure.

In the event of inconsistencies or contradictions between portions of the incorporated references and this application, the information in the preceding description shall control. The preceding description, given in order to enable one of ordinary skill in the art to practice the claimed disclosure, is not to be construed as limiting the scope of the disclosure, which is defined by the claims.

## Claims

1. An applicator 100 for applying a substance to a surface, the applicator comprising:
a hollow body 110, wherein the hollow body includes a distal portion112, a proximal portion114, an outer surface 118, an inner surface 116, and an activation region 120 between the distal portion and proximal portion, **characterized in that**
the hollow body includes two longitudinal grooves 122 on at least one of the outer surface 118 and the inner surface 116 in the activation region 120; and
wherein the hollow body has a first radial groove 124 in the activation region 120 that is on at least one of the outer surface 118 and the inner surface 116 and wherein the two longitudinal grooves 122 and the radial groove 124 have a width and depth sufficient to impart the desired flexibility to the activation region 120 while not reducing the thickness of the material forming the hollow body 110 to the point where it is vulnerable to puncture.

2. The applicator of claim 1, wherein the longitudinal grooves 122 are at least 90° apart.

3. The applicator of claim 1, wherein the longitudinal grooves 122 are 180°apart.

4. The applicator of any one of claims 1-3, wherein the hollow body 110 includes at least six longitudinal grooves 122 on the outer surface 118 in the activation region 120.

5. The applicator of claim 1, wherein the radial groove 124 extends at least 90° around the body.

6. The applicator of claim 1, wherein the radial groove 124 extends 180° around the body.

7. The applicator of any one of claims 1-6, wherein the hollow body 110 includes at least five radial grooves 124 on the outer surface 118 in the activation region 120.

8. The applicator of any one of claims 1-7, wherein the applicator 100 further comprises a cap 130, a spacer 170, a head 140, a frangible ampoule 160, and an absorbent component.

9. The applicator of any one of claims 1-8, wherein the applicator 100 comprises a polymer selected from the group consisting of a polyethylene, a linear low-density polyethylene, a medium-density polyethylene, a high-density polyethylene, a branched polyethylene, a polypropylene, and combinations thereof.

10. The applicator of any one of claims 1-9, wherein the applicator body 110 comprises a combination of linear low-density polyethylene and medium-density polyethylene.

## Patentansprüche

1. Ein Applikator 100 zum Aufbringen einer Substanz auf eine Oberfläche, der Applikator aufweisend:
einen Hohlkörper 110, wobei der Hohlkörper einen distalen Abschnitt 112, einen proximalen Abschnitt 114, eine Außenoberfläche 118, eine Innenoberfläche 116 und einen Aktivierungsbereich 120 zwischen dem distalen Abschnitt und dem proximalen Abschnitt aufweist, **dadurch gekennzeichnet, dass**
der Hohlkörper zwei Längsrillen 122 auf mindestens einer der Außenoberfläche 118 und der Innenoberfläche 116 in dem Aktivierungsbereich 120 aufweist; und
wobei der Hohlkörper eine erste radiale Rille 124 in dem Aktivierungsbereich 120 aufweist, die sich auf mindestens einer der Außenoberfläche 118 und der Innenoberfläche 116 befindet, und wobei die zwei Längsrillen 122 und die radiale Rille 124 eine ausreichende Breite und Tiefe aufweisen, um dem Aktivierungsbereich 120 die gewünschte Flexibilität zu verleihen, während die Dicke des Materials, das den Hohlkörper 110 ausbildet, nicht zu dem Punkt verringert wird, an dem es anfällig für ein Durchstechen ist.

2. Der Applikator nach Anspruch 1, wobei die Längsrillen 122 um mindestens 90° voneinander beabstandet sind.

3. Der Applikator nach Anspruch 1, wobei die Längsrillen 122 um 180° voneinander beabstandet sind.

4. Der Applikator nach einem der Ansprüche 1 bis 3, wobei der Hohlkörper 110 mindestens sechs Längsrillen 122 auf der Außenoberfläche 118 in dem Aktivierungsbereich 120 aufweist.

5. Der Applikator nach Anspruch 1, wobei sich die radiale Rille 124 um mindestens 90° um den Körper herum erstreckt.

6. Der Applikator nach Anspruch 1, wobei sich die radiale Rille 124 um 180° um den Körper herum erstreckt.

7. Der Applikator nach einem der Ansprüche 1 bis 6, wobei der Hohlkörper 110 mindestens fünf radiale Rillen 124 auf der Außenoberfläche 118 in dem Aktivierungsbereich 120 aufweist.

8. Der Applikator nach einem der Ansprüche 1 bis 7, wobei der Applikator 100 ferner eine Kappe 130, einen Abstandshalter 170, einen Kopf 140, eine zerbrechliche Ampulle 160 und eine Absorptionskomponente aufweist.

9. Der Applikator nach einem der Ansprüche 1 bis 8, wobei der Applikator 100 ein Polymer aufweist, das aus der Gruppe ausgewählt ist, bestehend aus einem Polyethylen, einem Polyethylen niedriger Dichte, einem Polyethylen mittlerer Dichte, einem Polyethylen hoher Dichte, einem verzweigten Polyethylen, einem Polypropylen und Kombinationen davon.

10. Der Applikator nach einem der Ansprüche 1 bis 9, wobei der Applikatorkörper 110 eine Kombination aus linearem Polyethylen niedriger Dichte und Polyethylen mittlerer Dichte aufweist.

## Revendications

1. Applicateur 100 pour appliquer une substance sur une surface, l'applicateur comprenant :
un corps creux110, dans lequel le corps creux comporte une partie distale 112, une partie proximale 114, une surface externe 118, une surface interne 116 et une région d'activation 120 entre la partie distale et la partie proximale, **caractérisé en ce que**
le corps creux comporte deux rainures longitudinales 122 sur au moins l'une de la surface externe 118 et de la surface interne 116 dans la région d'activation 120 ; et
dans lequel le corps creux a une première rainure radiale 124 dans la région d'activation 120 qui est sur au moins une de la surface externe 118 et de la surface interne 116 et dans lequel les deux rainures longitudinales 122 et la rainure radiale 124 ont une largeur et une profondeur suffisantes pour conférer la flexibilité souhaitée à la région d'activation 120 tout en ne réduisant pas l'épaisseur du matériau formant le corps creux 110 au point où elle est vulnérable à la perforation.

2. Applicateur selon la revendication 1, dans lequel les rainures longitudinales 122 sont espacées d'au moins 90°.

3. Applicateur selon la revendication 1, dans lequel les rainures longitudinales 122 sont espacées de 180°.

4. Applicateur selon l'une quelconque des revendications 1 à 3, dans lequel le corps creux 110 comprend au moins six rainures longitudinales 122 sur la surface extérieure 118 dans la région d'activation 120.

5. Applicateur selon la revendication 1, dans lequel la rainure radiale 124 s'étend d'au moins 90° autour du corps.

6. Applicateur selon la revendication 1, dans lequel la rainure radiale 124 s'étend à 180° autour du corps.

7. Applicateur selon l'une quelconque des revendications 1 à 6, dans lequel le corps creux 110 comprend au moins cinq rainures radiales 124 sur la surface extérieure 118 dans la région d'activation 120.

8. Applicateur selon l'une quelconque des revendications 1 à 7, dans lequel l'applicateur 100 comprend en outre un capuchon 130, une entretoise 170, une tête 140, une ampoule cassante 160, et un composant absorbant.

9. Applicateur selon l'une quelconque des revendications 1 à 8, dans lequel l'applicateur 100 comprend un polymère choisi dans le groupe constitué par un polyéthylène, un polyéthylène linéaire basse densité, un polyéthylène moyenne densité, un polyéthylène haute densité, un polyéthylène ramifié, un polypropylène, et des combinaisons de ceux-ci.

10. Applicateur selon l'une quelconque des revendications 1 à 9, dans lequel le corps d'applicateur 110 comprend une combinaison de polyéthylène linéaire basse densité et de polyéthylène moyenne densité.
